# EUROPEAN PATENT APPLICATION

(11) **EP 4 846 940 A1**
(43) Date of publication of application: **02.12.2026**
(21) Application number: 25819339.0
(22) Date of filing: 07.07.2025
(51) Int. Cl.: A61B 6/00, A61B 6/03

(54) **COORDINATE CALIBRATION DEVICE AND METHOD BASED ON DISTRIBUTED RAY SOURCE IMAGING SYSTEM**

(30) Priority: 05.11.2024 CN 202411562492
(71) Applicant: Nuray Technology Co., Ltd., Jintan Changzhou Jiangsu 213200 (CN)
(72) Inventor: JIN, Xin, Changzhou, Jiangsu 213200 (CN); TANG, Huaping, Changzhou, Jiangsu 213200 (CN); PAN, Jinsong, Changzhou, Jiangsu 213200 (CN); XIE, Dehua, Changzhou, Jiangsu 213200 (CN); FANG, Runze, Changzhou, Jiangsu 213200 (CN)
(74) Representative: Dilg, Haeusler, Schindelmann Patentanwaltsgesellschaft mbH
(86) International application number: PCT/CN2025/107312
(87) International publication number: WO 2026/097914

(57) **Abstract**

The present disclosure provides a coordinate calibration device and method based on a distributed X-ray source imaging system. The coordinate calibration device includes an X-ray source and a detector; the X-ray source is configured to emit X-rays; the detector includes a detecting portion and a marker portion; the detecting portion is configured to be disposed in a target region and on a side of an object to be detected opposite to the X-ray source, and the detecting portion is configured to receive the X-rays; the marker portion is disposed between the detecting portion and the X-ray source, and a relative position between the marker portion and the detecting portion is fixed; the marker portion is provided with at least two spaced marker points for a coordinate calibration of X-ray imaging; where the coordinate calibration is performed using the same dataset with the X-ray imaging. Known parameters of the marker portion are used to correct the source-detector relative coordinates, and the mechanical positioning advantage of the system is leveraged to assist in calculating the source-detector relative coordinates, so that the computational load of coordinate estimation is reduced. This may improve the relative positional accuracy or achieve a real-time position calibration, thereby avoiding secondary exposure and achieving convenient three-dimensional imaging.

## Description

### TECHNICAL FIELD

The present disclosure relates to the field of medical clinical diagnosis, and in particular, to a coordinate calibration device and method based on a distributed X-ray source imaging system, and an imaging system.

### BACKGROUND

Digital tomosynthesis (DT) is a type of X-ray imaging method. Its characteristic is that only a small number of projection angles are required to obtain tomographic images parallel to a detector direction, thereby allowing acquisition of depth information inside an object. Traditional digital radiography (DR) produces superimposed images of object structures, and easily leads to misdiagnosis when a lesion is overlapped. In contrast, DT may acquire internal depth information of the object and improve diagnostic accuracy. Compared with traditional CT, DT has advantages of lower radiation dose and higher resolution. In a DT system, a relative geometric accuracy between an X-ray source and a detector is crucial for a quality of a reconstructed image.

Common geometric calibration methods include visible-light calibration, phantom-based calibration, and triangulation calibration. Visible-light calibration is to derive relative coordinates between the detector and the X-ray source according to the principles of structured light. Phantom-based geometric calibration is to determine source-detector relative coordinates according to a feature of a specific calibration material embedded in a phantom in a projection image. Triangulation calibration is to employ signal transceivers (e.g., radio frequency transceivers or electromagnetic coils) in combination with corresponding signal sensing devices to compute a source-detector relative relationship.

Existing phantom-based calibration methods are designed for coordinate calibration of mobile single-point source systems, which require calculating source-detector spatial relative coordinates for each point source, resulting in a large amount of computation and low efficiency.

### SUMMARY

To solve the above technical problems, embodiments of the present disclosure provide a coordinate calibration device and method based on a distributed X-ray source imaging system, and an imaging system, which may improve the relative position accuracy or achieve a real-time position calibration, thereby avoiding secondary exposure and achieving more convenient three-dimensional imaging.

According to an aspect of the inventive concept of the present disclosure, a coordinate calibration device based on a distributed X-ray source imaging system is provided, including: an X-ray source configured to emit X-rays; and a detector, including: a detecting portion configured to be disposed on a side of an object to be detected opposite to the X-ray source, where the detecting portion is configured to receive the X-rays to image the object to be detected; and a first marker portion disposed between the detecting portion and the X-ray source, where the first marker portion includes a region of interest and a non-region of interest outside the region of interest, an image of the object to be detected after exposure is located in the region of interest, the first marker portion is provided with a plurality of first marker points having three-dimensional geometric shapes, and images of the plurality of first marker points after exposure are located in the first marker portion for a coordinate calibration of X-ray imaging; where the coordinate calibration includes calculating a relative positional relationship between the detecting portion and the X-ray source by using the shapes of the plurality of first marker points and imaging shapes of the plurality of first marker points, and the coordinate calibration is performed using the same dataset with X-ray scanning and three-dimensional imaging of the target object.

According to some embodiments of the present disclosure, each of the first marker points includes a marker symbol or a marker pattern, and different marker symbols or marker patterns have a predetermined relative positional relationship.

According to some embodiments of the present disclosure, the image of at least one first marker point after exposure is located in the non-region of interest for the coordinate calibration of X-ray imaging; and/or the image of at least one first marker point after exposure is located in the region of interest for the coordinate calibration of X-ray imaging.

According to some embodiments of the present disclosure, the first marker points are dot-shaped, filament-shaped, strip-shaped, L-shaped columns, or S-shaped columns.

According to some embodiments of the present disclosure, the number of the first marker points is less than or equal to four.

According to some embodiments of the present disclosure, the first marker points are embedded into a top portion of the first marker portion on a side away from the detecting portion.

According to some embodiments of the present disclosure, the detecting portion and the first marker portion are located on the same side of the object to be detected, and the object to be detected, the first marker portion, and the detecting portion are sequentially disposed in close contact.

According to some embodiments of the present disclosure, the coordinate calibration device further includes: a first support having one end connected to the detecting portion and the other end connected to the X-ray source, where the first support is a rigid structure configured to maintain the relative positional relationship between the detecting portion and the X-ray source unchanged.

According to some embodiments of the present disclosure, the coordinate calibration device further includes: a second support having one end connected to the detecting portion and the other end connected to the first marker portion, where the detecting portion and the first marker portion are respectively disposed on opposite sides of the object to be detected, and the second support is a rigid structure configured to maintain a relative positional relationship between the detecting portion and the first marker portion unchanged.

According to some embodiments of the present disclosure, the other end of the second support extends in a direction away from the detecting portion and is connected to the X-ray source, and the second support is configured to maintain a relative positional relationship among the X-ray source, the detecting portion, and the first marker portion unchanged.

According to some embodiments of the present disclosure, the first marker portion and the detecting portion are respectively located on opposite sides of the object to be detected, and the coordinate calibration device further includes: a third support having one end connected to the first marker portion and the other end connected to the X-ray source, where the third support is a rigid structure configured to maintain a relative positional relationship between the first marker portion and the X-ray source unchanged.

According to some embodiments of the present disclosure, the coordinate calibration device further includes: a second marker portion disposed between the detecting portion and the object to be detected, where opposite sides of the second marker portion are respectively in close contact with the object to be detected and the detecting portion, and a second marker point is embedded into a top portion of the second marker portion on a side away from the detecting portion.

According to some embodiments of the present disclosure, the coordinate calibration device further includes: a second marker portion disposed on a side of the object to be detected opposite to the detecting portion, where a second marker point is embedded into a top portion of the second marker portion on a side away from the detecting portion; and a fourth support having one end connected to the detecting portion and the other end connected to the second marker portion, where the fourth support is a rigid structure configured to maintain a relative positional relationship between the detecting portion and the second marker portion unchanged.

According to some embodiments of the present disclosure, a thickness of the first marker portion and a thickness of the second marker portion range from 1 mm to 3 mm or from 2 mm to 15 mm.

According to some embodiments of the present disclosure, the X-ray source is a distributed multi-point source array.

According to some embodiments of the present disclosure, both ends of the first support are respectively connected to the detecting portion and the X-ray source by magnetic attraction; and/or both ends of the second support are respectively connected to the detecting portion and the first marker portion by magnetic attraction; and/or both ends of the third support are respectively connected to the first marker portion and the X-ray source by magnetic attraction; and/or both ends of the fourth support are respectively connected to the detecting portion and the second marker portion by magnetic attraction.

According to some embodiments of the present disclosure, the relative positional relationship between the detecting portion and the X-ray source includes a tilt angle and an offset angle of the detecting portion relative to the X-ray source as well as a distance between the detecting portion and the X-ray source.

According to some embodiments of the present disclosure, the object to be detected is a tooth, a torso, or a whole human body.

According to another aspect of the inventive concept of the present disclosure, an imaging system is provided, including: the coordinate calibration device described above; a data conversion device configured to convert a detection signal generated by a detecting portion of the coordinate calibration device into digital data; and an image processing device configured to perform imaging according to the digital data.

According to yet another aspect of the inventive concept of the present disclosure, a coordinate calibration method based on a distributed X-ray source imaging system is provided, applicable to the coordinate calibration device described above, the coordinate calibration method including: step S01 of determining a relative position of at least one of a detecting portion and an X-ray source with respect to a first marker portion; step S02 of fixing the detecting portion in a target region so that the detecting portion and the X-ray source are located on opposite sides of an object to be detected; step S03 of directing X-rays emitted from the X-ray source to the detecting portion; and step S04 of performing a coordinate calibration on image data acquired by the detecting portion according to relative positional data of at least one of the detecting portion and the X-ray source with respect to the first marker points on the first marker portion; where the first marker points have three-dimensional geometric shapes, and spatial coordinates of the X-ray source and spatial coordinates of the detecting portion are calculated using the shapes of the first marker points and imaging coordinates of the first marker points, to obtain a relative positional relationship between the X-ray source and the detecting portion.

According to some embodiments of the present disclosure, step S01 includes: fixing the detecting portion in the target region, so that the detecting portion and the first marker portion are located on the same side of the object to be detected, and the object to be detected, the first marker portion, and the detecting portion are sequentially disposed in close contact; or fixing the detecting portion in the target region, so that the first marker portion and the detecting portion are respectively located on opposite sides of the object to be detected.

According to some embodiments of the present disclosure, the relative positional relationship between the X-ray source and the detecting portion is represented by a functional relationship between spatial coordinates of focal spots of the X-ray source and spatial coordinates of pixels of the detecting portion.

According to some embodiments of the present disclosure, the focal spots of the X-ray source are obtained by: step S11 of establishing a spatial coordinate system with a center point of the detecting portion as an origin, defining coordinates of a first focal spot of the X-ray source as (x, y, z), and deriving spatial coordinates of the first marker point according to coordinates of the X-ray source or coordinates of the detecting portion; step S12 of simulating an emitted beam according to a geometric relationship among the coordinates of the first focal spot, the first marker points, and the detecting portion, to generate a simulated projection image; step S13 of extracting features of the first marker points in the simulated projection image, defined as simulated features *fᵢ*(*x, y, z*) ,(i=1,2,...,n); and calculating features of the corresponding first marker points in a real projection image, defined as *gᵢ*(*x, y, z*),(i=1,2,...,n); step S14 of obtaining simulated coordinates of the first focal spot by solving an objective function, where the objective function is: ${Argmin}_{x , y , z} \left({\sum }_{i = 1}^{n} norm \left({f}_{i}\left(x, y, z\right)-{g}_{i}\left(x, y, z\right)\right)\right)$; and step S15 of sequentially switching to all remaining focal spots of the X-ray source, and repeatedly performing steps S11 to S14 until the simulated coordinates of all focal spots are calculated.

In the coordinate calibration device based on a distributed X-ray source imaging system according to embodiments of the present disclosure, known parameters of a marker portion are used to correct the source-detector relative coordinates. By using the shapes and images of the marker points, the tilt angle and offset angle of the marker portion may be calculated to correct the imaging of the detector. Further, by leveraging the mechanical positioning advantage of the system to assist in calculating the source-detector relative coordinates, the computational load of coordinate estimation is reduced. This may improve the relative positional accuracy or achieve a real-time position calibration, thereby avoiding secondary exposure and achieving more convenient three-dimensional imaging.

### BRIEF DESCRIPTION OF THE DRAWINGS

FIG. 1 shows a schematic structural diagram of a coordinate calibration device based on a distributed X-ray source imaging system according to an exemplary embodiment of the present disclosure;
FIG. 2 shows a schematic structural diagram of a coordinate calibration device based on a distributed X-ray source imaging system according to another exemplary embodiment of the present disclosure;
FIG. 3 shows a schematic structural diagram of a coordinate calibration device based on a distributed X-ray source imaging system according to yet another exemplary embodiment of the present disclosure;
FIG. 4 shows a schematic structural diagram of a coordinate calibration device based on a distributed X-ray source imaging system according to still another exemplary embodiment of the present disclosure;
FIG. 5 shows a schematic structural diagram of a coordinate calibration device based on a distributed X-ray source imaging system according to a further exemplary embodiment of the present disclosure;
FIG. 6 shows a schematic structural diagram of a coordinate calibration device based on a distributed X-ray source imaging system according to another further exemplary embodiment of the present disclosure;
FIG. 7 shows a schematic structural diagram of a coordinate calibration device based on a distributed X-ray source imaging system according to yet another further exemplary embodiment of the present disclosure;
FIG. 8 shows a schematic structural diagram of a coordinate calibration device based on a distributed X-ray source imaging system applied to whole-body scanning according to another exemplary embodiment of the present disclosure;
FIG. 9 shows a flowchart of a coordinate calibration method based on a distributed X-ray source imaging system according to an exemplary embodiment of the present disclosure; and
FIG. 10 shows a flowchart of steps for obtaining focal spots of the X-ray source in the coordinate calibration method based on a distributed X-ray source imaging system according to an exemplary embodiment of the present disclosure.

Reference numerals:
1 - detecting portion; 2 - X-ray source; 3 - first marker portion; 3' - second marker portion; 4 - first marker point; 4' - second marker point; 5 - first support; 6 - second support; 7 - third support; 8 - fourth support; 9 - coordinate calibration device.

### DETAILED DESCRIPTION OF EMBODIMENTS

To make objectives, technical solutions, and advantages of the present disclosure clearer, the present disclosure will be further described in detail below with reference to specific embodiments and the accompanying drawings.

However, it should be understood that these descriptions are merely exemplary and are not intended to limit the scope of the present disclosure. In the following detailed description, for the convenience of explanation, many specific details are set forth to provide a comprehensive understanding of the embodiments of the present disclosure. However, it is obvious that one or more embodiments may also be implemented without these specific details. In addition, in the following description, descriptions of known technologies are omitted to avoid unnecessarily confusing the concept of the present disclosure.

The terms used herein are merely for describing specific embodiments and are not intended to limit the present disclosure. The term "including" as used herein indicates the presence of features, steps, and operations, but does not exclude the presence or addition of one or more other features, steps, and operations.

When expressions such as "at least one of A, B, and C" are used, they should generally be interpreted in the sense commonly understood by those skilled in the art (for example, "a system having at least one of A, B, and C" should include but not be limited to a system having A alone, a system having B alone, a system having C alone, a system having both A and B, a system having both A and C, a system having both B and C, and/or a system having all of A, B, and C). When expressions such as "at least one of A, B, or C" are used, they should generally be interpreted in the sense commonly understood by those skilled in the art (for example, "a system having at least one of A, B, or C" should include but not be limited to a system having A alone, a system having B alone, a system having C alone, a system having both A and B, a system having both A and C, a system having both B and C, and/or a system having all of A, B, and C).

All terms used herein (including technical and scientific terms) have the meanings commonly understood by those skilled in the art unless otherwise defined. It should be noted that the terms used herein should be interpreted as having meanings consistent with the context of this specification, and should not be interpreted in an idealized or overly rigid manner.

In an embodiment of the present disclosure, known parameters of a phantom are used to correct source-detector relative coordinates. In some exemplary embodiments of the present disclosure, for a coordinate calibration of a distributed multi-point source system, since the relative coordinate accuracy between point sources in the distributed point source system is high, it is possible to leverage the mechanical positioning advantage of the system to assist in calculating the source-detector relative coordinates, thereby significantly reducing the computational load of coordinate estimation.

Embodiments of the present disclosure mainly propose an innovative calibration solution for the source-detector relative position in a distributed multi-point source system. Since the point sources in such a distributed multi-point source system have fixed positions, this prior information may be fully utilized to determine the source-detector relative positional relationship. On the other hand, a detecting portion usually needs to be disassembled, installed, and positioned in a target region, and a support or similar devices is generally provided between the detecting portion and the point sources to determine the relative positional relationship. However, the source-detector relative positioning accuracy achieved by mechanical structures is typically insufficient. For example, in two-dimensional fluoroscopic imaging such as dental X-ray machines, this does not affect the image clarity; however, in three-dimensional tomographic imaging, which requires synthesizing data from multiple viewing angles, insufficient positioning accuracy may significantly degrade the quality of 3D reconstruction. In addition, during disassembly and reinstallation of the detecting portion, coordinate errors are usually introduced. Therefore, a calibration method is still required to correct the relative coordinates between the point sources and the detecting portion in order to improve the relative positional accuracy or to achieve a real-time position calibration. Meanwhile, embodiments of the present disclosure further propose the use of a detecting portion with special markers, and fewer marker points, such as one or two marker points, are used to complete both the calibration of the source-detector relative position and the scanning imaging in a single exposure. This avoids the need for a second exposure, enables more convenient three-dimensional imaging, and also corrects for the effects caused by offset/tilt of the marker portion that may occur during attachment and fixation.

Embodiments of the present disclosure provide a coordinate calibration device and method based on a distributed X-ray source imaging system, as well as an imaging system, which may improve the relative position accuracy or achieve real-time position calibration, thereby avoiding secondary exposure and achieving more convenient three-dimensional imaging.

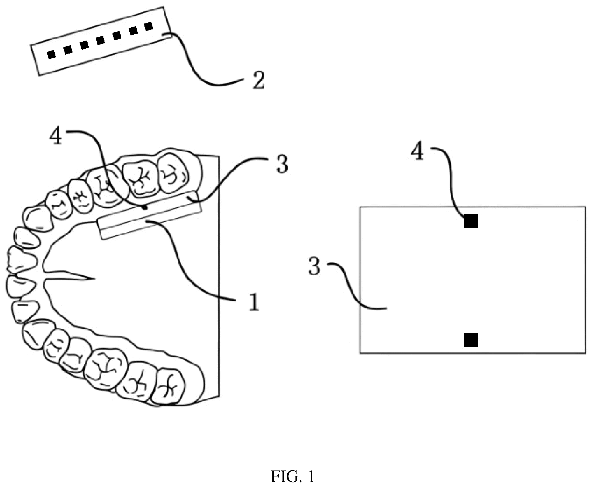

FIG. 1 shows a schematic structural diagram of a coordinate calibration device based on a distributed X-ray source imaging system according to an exemplary embodiment of the present disclosure.

According to an aspect of the inventive concept of the present disclosure, a coordinate calibration device based on a distributed X-ray source imaging system is provided. As shown in FIG. 1, the coordinate calibration device may include a detector and an X-ray source 2, and the detector includes a detecting portion 1 and a marker portion 3. In this embodiment, the marker portion 3 is configured to calibrate a relative positional relationship between the X-ray source 2 and the detecting portion 1. Therefore, the marker portion 3 may be regarded as a coordinate calibration device or a part thereof.

For example, in an embodiment of the present disclosure, the X-ray source 2 may be a distributed X-ray source that includes a plurality of target spots, such as a plurality of X-ray target spots. For example, as shown in FIG. 1 to FIG. 7, a plurality of small rectangular dark spots in the X-ray source 2 may be regarded as a plurality of target spots of the distributed X-ray source. In a distributed X-ray source, a target spot refers to an emission spot or focal spot of the X-ray source 2. Specifically, high-energy electrons are emitted from a cathode and bombard a metal anode target to generate X-rays. The energy of the emitted X-rays depends on the material of the anode target, while the intensity of the X-rays depends on the current and energy of the electrons bombarding the anode target. In a distributed X-ray source, multiple cathodes correspond one-to-one with multiple target spots, so that the multiple target spots receive electron beams from the multiple cathodes to generate multiple X-ray beams. Such a design allows the distributed X-ray source to generate more X-ray radiation sources with fewer cathode components, thereby improving system stability, reducing the number of cathode components required, and lowering production costs of the apparatus.

In an imaging system using a distributed X-ray source, multiple target spots may be combined together and sequentially activated at different angles according to a preset order, thereby acquiring multiple projection datasets from different angles. These projection datasets may be used in computer reconstruction algorithms to generate high-quality cross-sectional images. One advantage of using a distributed X-ray source is the reduction of artifacts and the improvement of image quality. By employing multiple target spots, the emission positions of X-ray beams are more evenly distributed, providing more projection angles and data, which may reduce artifacts in reconstructed images and provide more accurate anatomical information. In other words, in a distributed X-ray source, the target spots refer to the points or regions from which X-ray beams are emitted, and their distribution helps to obtain high-quality projection data for image reconstruction.

In an embodiment of the present disclosure, a plurality of target spots in a distributed X-ray source may be arranged in a predetermined first direction. For example, the predetermined first direction may be a linear direction or an arcuate direction. The embodiments of the present disclosure do not specifically limit the arrangement of target spots in the distributed X-ray source.

In some exemplary embodiments of the present disclosure, the detecting portion 1 may include a plurality of detecting units arranged in one dimension or two dimensions.

For example, a detecting unit may include at least one detector crystal. In one case, a detecting unit may include a single detector crystal. In another case, a detecting unit may include a plurality of detector crystals, which may be arranged in a one-dimensional direction or in a two-dimensional array.

It should be understood that each detector crystal is a basic unit of the detector, which may absorb radiation (such as X-rays) and convert it into other forms of energy, such as optical signals or electrical signals. For example, a material of the detector crystal may include oxides and halides (e.g., iodides and fluorides). According to some optional embodiments of the present disclosure, the coordinate calibration device may be applied to calibration of a dental tomographic imaging system. It should be noted that, although a dental tomographic imaging system is described herein as an example to illustrate various concepts of the present disclosure, the embodiments of the present disclosure are not limited thereto. Without departing from the spirit and principles of the present disclosure, the coordinate calibration device provided in embodiments of the present disclosure may also be applied to other scanning imaging systems.

According to some embodiments of the present disclosure, the detecting portion 1 is configured to be placed in a target region and arranged on a side of an object to be detected, while the X-ray source 2 is arranged on an opposite side of the object to be detected. The detecting portion 1 is configured to receive radiation such as X-rays to image the object to be detected. For example, the object to be detected may be a tooth, and the target region may be inside an oral cavity.

According to some optional embodiments of the present disclosure, the detecting portion 1 and the first marker portion 3 may be disposed on the same side of the object to be detected, and the object to be detected, the first marker portion 3, and the detecting portion 1 are sequentially arranged in close contact. The detecting portion 1 is configured to be placed inside the oral cavity in close contact with the tooth to be imaged. It should be noted that the term "close contact " herein includes the following situations: the detecting portion 1 is in direct contact with an inner surface of the tooth; or the detecting portion 1 is positioned in the oral cavity by a contact member such that the detecting portion 1 is arranged at a small distance or gap from the inner surface of the tooth.

According to some embodiments of the present disclosure, the first marker portion 3 may be disposed between the detecting portion 1 and the X-ray source 2, and the relative position between the first marker portion 3 and the detecting portion 1 is fixed.

According to some embodiments of the present disclosure, the first marker portion 3 includes a region of interest and a non-region of interest outside the region of interest. An image of the object to be detected after exposure is located in the region of interest. The first marker portion 3 is provided with a plurality of first marker points 4 having three-dimensional geometric shapes, and images of the first marker points 4 after exposure are located on the first marker portion 3 for coordinate calibration of X-ray imaging. The coordinate calibration includes calculating a relative positional relationship between the detecting portion 1 and the X-ray source 2 by using the shapes of the plurality of first marker points 4 and imaging shapes of the plurality of first marker points 4.

According to some embodiments of the present disclosure, the coordinate calibration is performed using the same dataset with the X-ray imaging.

According to some embodiments of the present disclosure, each first marker point 4 includes a marker symbol or a marker pattern, and different marker symbols or marker patterns have a predetermined relative positional relationship.

It should be noted that in this embodiment, the marker symbols may be English letters, Greek letters, Arabic numerals, or the like, and the marker patterns may be circles, squares, triangles, or the like.

According to some embodiments of the present disclosure, the image of at least one first marker point 4 after exposure is located in the non-region of interest for the coordinate calibration of X-ray imaging.

According to some embodiments of the present disclosure, the image of at least one first marker point 4 after exposure is located in the region of interest for the coordinate calibration of X-ray imaging.

It should be noted that in this embodiment, when the image of the first marker point 4 is located in the region of interest, the first marker point 4 overlaps with the exposure image of the object to be detected, and the image of the first marker point 4 needs to be removed from the exposure image of the object to be detected. Specifically, the image of the first marker point 4 may be removed from the exposure image by extracting the first marker point 4 from the exposure image of the object to be detected and performing interpolation.

According to some embodiments of the present disclosure, the relative positional relationship between the detecting portion 1 and the X-ray source 2 includes a tilt angle and an offset angle of the detecting portion 1 relative to the X-ray source 2, as well as a distance between the detecting portion 1 and the X-ray source 2.

In this embodiment, known parameters of the marker portion 3 are used to calibrate the source-detector relative coordinates. By using the shapes of the first marker points 4 and the imaging coordinates of the first marker points 4, spatial coordinates of the X-ray source 2 and the detecting portion 1 may be calculated to obtain the relative positional relationship between the X-ray source 2 and the detecting portion 1. The imaging of the detecting portion 1 is then corrected. By using the mechanical positioning advantage of the system to assist in calculating the source-detector relative coordinates, the computational load of coordinate estimation may be reduced. This may improve the relative positional accuracy or achieve real-time position calibration, thereby avoiding secondary exposure and achieving more convenient intraoral three-dimensional imaging.

According to some optional embodiments of the present disclosure, the first marker points 4, the first marker portion 3, and the detecting portion 1 may be tightly bound and inseparable. The first marker portion 3 and the detecting portion 1 may be fixedly connected, for example, the two may be adhered together, and the first marker points 4 are fixedly arranged on the first marker portion 3.

According to some embodiments of the present disclosure, the first marker points 4 are configured to correct geometric errors caused by disassembly and movement of the detecting portion 1.

According to some optional embodiments of the present disclosure, the first marker points 4 are arranged at a top portion of the first marker portion 3 on a side away from the detecting portion 1. A thickness of the first marker portion 3 is a known parameter, i.e., a distance between the first marker points 4 and the detecting portion 1 is a known parameter. Because of the fixed connection between the first marker portion 3 and the detecting portion 1, the distance between the first marker points 4 and the detecting portion 1 remains constant, and does not change due to, for example, different positions of the first marker portion 3 and the detecting portion 1 in the target oral cavity.

According to some alternative embodiments of the present disclosure, the first marker portion 3 is detachably mounted on the detecting portion 1. In other words, a set of first marker points 4 and a first marker portion 3 may correspond to a detecting portion 1, or a set of first marker points 4 and a first marker portion 3 may be removed from a detecting portion module and installed onto another detecting portion 1 to form a new detecting portion module. This improves applicability and facilitates subsequent cleaning, disinfection, and sterilization. According to some optional embodiments of the present disclosure, the coordinate calibration device is applicable to a medical imaging system. In further optional embodiments, the coordinate calibration device is applicable to a dental tomographic imaging system.

According to some alternative embodiments of the present disclosure, the number of the first marker points 4 is less than or equal to four.

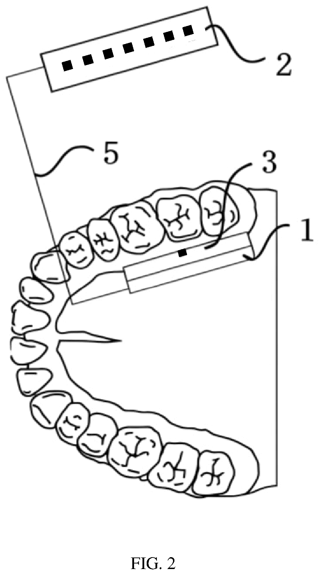

FIG. 2 shows a schematic structural diagram of a coordinate calibration device based on a distributed X-ray source imaging system according to another exemplary embodiment of the present disclosure.

According to some alternative embodiments of the present disclosure, as shown in FIG. 2, the coordinate calibration device further includes a first support 5. One end of the first support 5 is connected to the detecting portion 1, and the other end is connected to the X-ray source 2. The first support 5 is a rigid structure configured to maintain the relative position between the detecting portion 1 and the X-ray source 2 unchanged.

In this embodiment, the detecting portion 1 and the first support 5 are fixedly connected, and the X-ray source 2 and the first support 5 are fixedly connected, so as to ensure that the relative position and relative distance between the detecting portion 1 and the X-ray source 2 remain unchanged, thereby improving the calibration accuracy. Both ends of the first support 5 are respectively connected to the detecting portion 1 and the X-ray source 2 by magnetic attraction.

According to some optional embodiments of the present disclosure, the first marker points 4, the first marker portion 3, and the detecting portion 1 may be tightly bound and inseparable. The first marker portion 3 and the detecting portion 1 may be fixedly connected, for example, the two may be adhered together, and the first marker points 4 are fixedly arranged on the first marker portion 3. The thickness of the first marker portion 3 is a known parameter, i.e., the distance between the first marker points 4 and the detecting portion 1 is a known parameter. Because of the fixed connection between the first marker portion 3 and the detecting portion 1, the distance between the first marker points 4 and the detecting portion 1 remains constant, and does not change due to, for example, different positions of the first marker portion 3 and the detecting portion 1 in the target oral cavity.

According to some alternative embodiments of the present disclosure, the first marker portion 3 is detachably mounted on the detecting portion 1. In other words, a set of first marker points 4 and a first marker portion 3 may correspond to a detecting portion 1, or a set of first marker points 4 and a first marker portion 3 may be removed from a detecting portion module and installed onto another detecting portion 1 to form a new detecting portion module. This improves applicability and facilitates subsequent cleaning, disinfection, and sterilization.

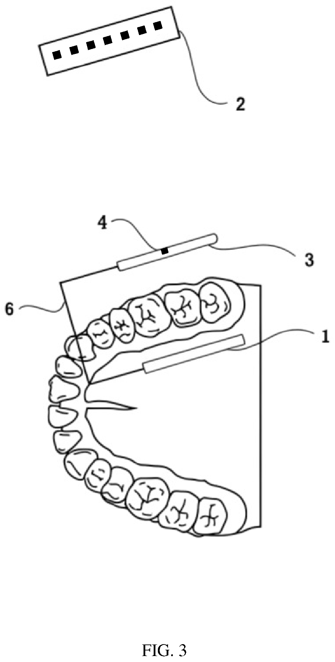

FIG. 3 shows a schematic structural diagram of a coordinate calibration device based on a distributed X-ray source imaging system according to yet another exemplary embodiment of the present disclosure.

According to some optional embodiments of the present disclosure, as shown in FIG. 3, the coordinate calibration device further includes a second support 6. One end of the second support 6 is connected to the detecting portion 1, and the other end is connected to the first marker portion 3, so that the detecting portion 1 and the first marker portion 3 are respectively located on opposite sides of the tooth to be imaged. The second support 6 is a rigid structure configured to maintain the relative position between the detecting portion 1 and the marker portion 3 unchanged.

In this embodiment, the X-ray source 2 is in a free state without being fixed to a marker module by a support. The first marker points 4 and the first marker portion 3 in the marker module are coupled through the rigid support to maintain their relative positional relationship unchanged.

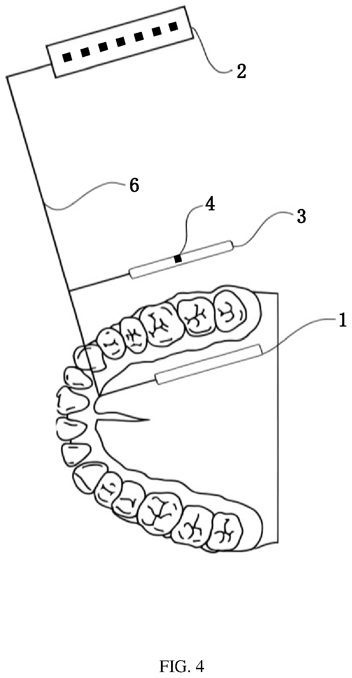

FIG. 4 shows a schematic structural diagram of a coordinate calibration device based on a distributed X-ray source imaging system according to still another exemplary embodiment of the present disclosure.

According to some alternative embodiments of the present disclosure, as shown in FIG. 4, the other end of the second support 6 extends in a direction away from the detecting portion 1 and is connected to the X-ray source 2. The second support 6 is configured to maintain the relative positions among the X-ray source 2, the detecting portion 1, and the first marker portion 3 unchanged.

In this embodiment, the first marker points 4 and the first marker portion 3 in the marker module are coupled with the detecting portion 1 through a rigid support to maintain their relative positional relationship. During the calibration and imaging process, only the effect of detachment of the detecting portion 1 on the coordinates needs to be considered, and the relative geometric stability is very high. Both ends of the second support 6 are respectively connected to the detecting portion 1 and the first marker portion 3 by magnetic attraction.

According to some embodiments of the present disclosure, the X-ray source 2 is a distributed multi-point source array.

According to some optional embodiments of the present disclosure, the first marker points 4 are dot-shaped, filament-shaped, strip-shaped, L-shaped columns, or S-shaped columns.

In this embodiment, when the detecting portion 1 is attached to the object to be detected, tilting or shifting may occur, which may lead to inaccurate imaging. Based on this, it is possible to calculate a tilt angle and an offset angle of the detecting portion 1 by using the shapes of the first marker points 4 and the imaging shapes of the first marker points 4, and then the imaging may be corrected according to the tilt angle and the offset angle.

According to some optional embodiments of the present disclosure, for example, if the first marker point 4 is a cube with a side length of a, then under normal conditions without tilting or shifting, the image of the first marker point 4 is a square of a x a. If the image of the first marker point 4 is a rectangle of √2a x a, it indicates that the detecting portion 1 has shifted by 45°. Based on this, the image of the object to be detected may be corrected and adjusted accordingly.

According to some embodiments of the present disclosure, as shown in FIG. 1 to FIG. 4, the detector and the first marker points 4 form a marker object, and spatial coordinates of the marker object may be derived according to coordinates of the detector.

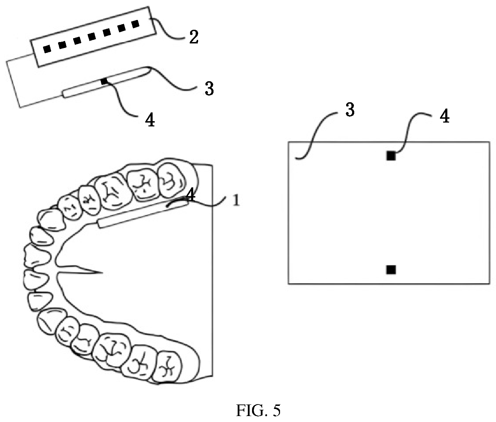

FIG. 5 shows a schematic structural diagram of a coordinate calibration device based on a distributed X-ray source imaging system according to a further exemplary embodiment of the present disclosure.

According to some embodiments of the present disclosure, as shown in FIG. 5, the first marker points 4 are bound to the X-ray source 2. A size and position of the first marker points 4 may be adjusted according to the source-detector relationship so that their images after exposure are located in the non-region of interest.

In this embodiment, the spatial coordinates of the marker object may be derived according to the coordinates of the X-ray source 2.

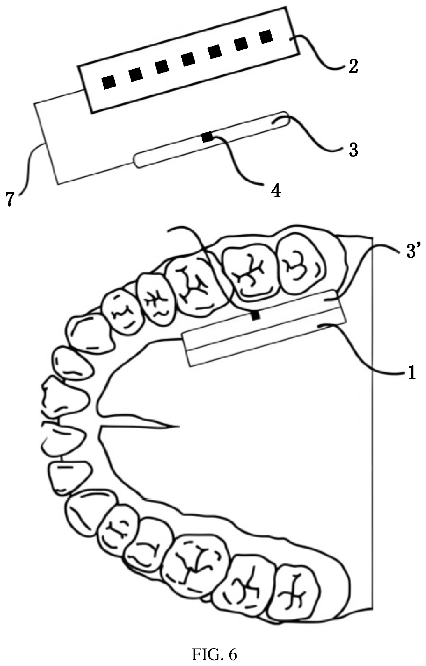

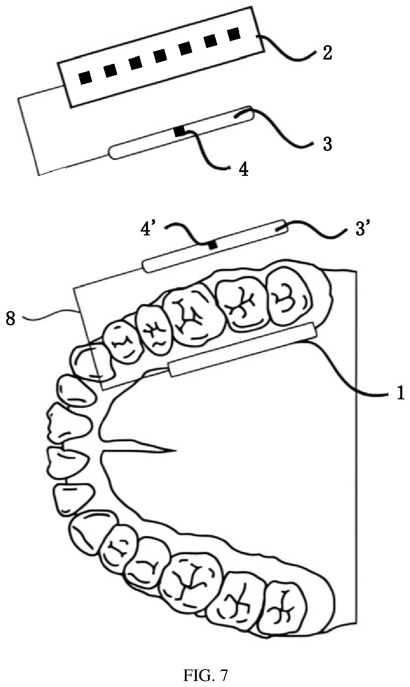

FIG. 6 shows a schematic structural diagram of a coordinate calibration device based on a distributed X-ray source imaging system according to another further exemplary embodiment of the present disclosure. FIG. 7 shows a schematic structural diagram of a coordinate calibration device based on a distributed X-ray source imaging system according to yet another further exemplary embodiment of the present disclosure.

According to some embodiments of the present disclosure, as shown in FIG. 6, the coordinate calibration device further includes a third support 7 and a second marker portion 3'. One end of the third support 7 is connected to the first marker portion 3, and the other end is connected to the X-ray source 2. The third support 7 is a rigid structure configured to maintain the relative position between the first marker portion 3 and the X-ray source 2 unchanged. The second marker portion 3' is located between the detecting portion 1 and the object to be detected, and opposite sides of the second marker portion 3' are respectively attached to the object to be detected and the detecting portion 1. A second marker point 4' is embedded into a top portion of the second marker portion 3' on a side away from the detecting portion 1. In other words, the first marker portion 3 and the second marker portion 3' are provided, which are respectively bound to the X-ray source 2 and the detecting portion 1. The first marker portion 3 and the second marker portion 3' are respectively provided with first marker points 4 and second marker points 4'.

In this embodiment, the spatial coordinates of the marker object may be derived according to the coordinates of the X-ray source 2 and the detector. The first marker point 4 (bound to the X-ray source 2) and the second marker point 4' (bound to the detecting portion 1) are defined. To address the problem of poor spatial resolution of the first marker point 4, only the centroid of the first marker point 4 may be used for calculation (i.e., discarding the center points of the edges). This solution not only solves coordinate errors in the source-detector distance direction, but also solves coordinate estimation errors caused by insufficient spatial resolution, thereby significantly improving the coordinate estimation accuracy.

According to some embodiments of the present disclosure, as shown in FIG. 7, the coordinate calibration device further includes a third support 7, a second marker portion 3', and a fourth support 8. The second marker portion 3' and the detecting portion 1 are disposed on opposite sides of the object to be detected. A second marker point 4' is embedded into a top portion of the second marker portion 3' on a side away from the detecting portion 1. One end of the fourth support 8 is connected to the detecting portion 1, and the other end is connected to the second marker portion 3'. The fourth support 8 is a rigid structure configured to maintain the relative position between the detecting portion 1 and the second marker portion 3' unchanged. Both ends of the third support 7 are respectively connected to the first marker portion 3 and the X-ray source 2 by magnetic attraction. Both ends of the fourth support 8 are respectively connected to the detecting portion 1 and the second marker portion 3' by magnetic attraction.

According to some embodiments of the present disclosure, the first marker points 4 may be installed between the detecting portion 1 and the tooth or between the tooth and the X-ray source, depending on the selection of two types of detecting portion modules. The detecting portion 1 is placed in a region that does not affect imaging of the tooth. When the first marker points 4 are placed between the detecting portion 1 and the tooth, a certain distance is formed between the first marker points 4 and the detecting portion 1, and the first marker portion 3 with a known thickness is inserted between them, so that the first marker points 4, the first marker portion 3, and the detecting portion 1 form an integral unit that is placed into or removed from the oral cavity together during scanning. When the first marker points 4 are installed between the tooth and the X-ray source, the detecting portion 1 and the first marker portion 3 may be connected by a rigid support to fix the relative coordinates between the detecting portion 1 and the first marker points 4.

According to some optional embodiments of the present disclosure, the first marker portion 3 and the second marker portion 3' are made of X-ray transmissive materials, such as plastic, PMMA, or the like.

According to some optional embodiments of the present disclosure, when the coordinate calibration device is applied to a dental tomographic imaging system, i.e., when the object to be detected is a tooth, the thickness of the first marker portion 3 and the thickness of the second marker portion 3' range from 1 mm to 3 mm. Alternatively, when the coordinate calibration device is applied to a general medical imaging system, i.e., when the object to be detected is a limb joint, the thickness of the marker portion 3 ranges from 2 mm to 15 mm.

According to some embodiments of the present disclosure, the implementations shown in FIG. 1 and FIG. 3 adopt a split structure. By separating the detecting portion 1 from the X-ray source 2, more detailed and accurate adjustments may be made for different application scenarios, which provides higher degrees of freedom, more flexible positioning, and easier accurate positioning to small or hidden objects to be detected.

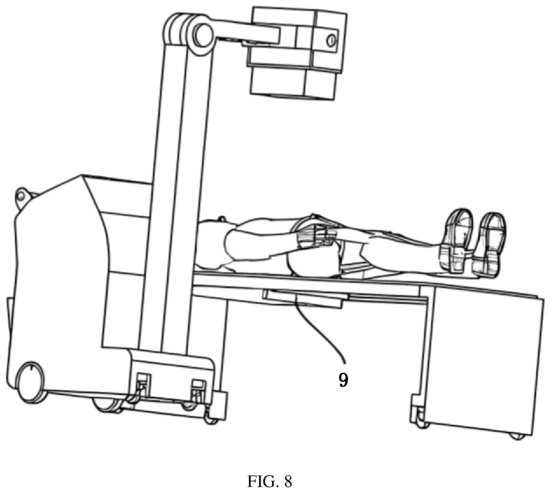

FIG. 8 shows a schematic structural diagram of a coordinate calibration device based on a distributed X-ray source imaging system applied to whole-body scanning according to another exemplary embodiment of the present disclosure.

According to some embodiments of the present disclosure, in the implementation shown in FIG. 8, the object to be detected is a tooth, a torso, or a whole human body. In other words, the coordinate calibration device 9 of this embodiment may also be applied to calibration of geometric coordinates for whole-body scanning. The coordinate calibration device 9 includes a detector having a plurality of first marker points 4 and/or a plurality of second marker points 4'.

According to another aspect of the inventive concept of the present disclosure, an imaging system is provided, including the above-mentioned coordinate calibration device, a data conversion device, and an image processing device.

According to some embodiments of the present disclosure, the data conversion device is configured to convert a detection signal generated by the detecting portion 1 of the coordinate calibration device into digital data.

According to some embodiments of the present disclosure, the image processing device is configured to perform imaging according to the digital data.

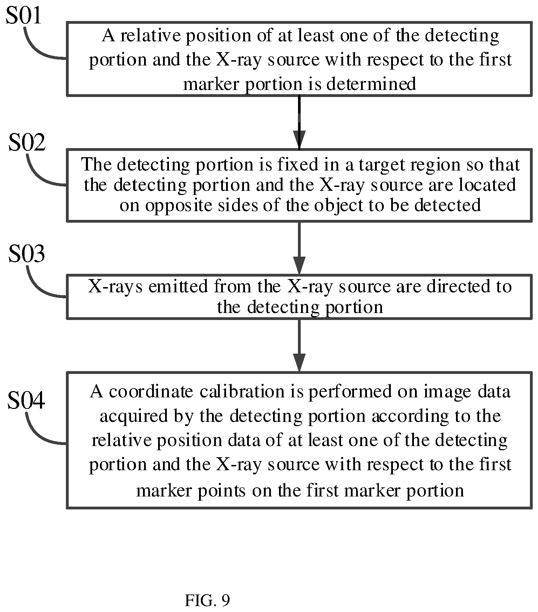

FIG. 9 shows a flowchart of a coordinate calibration method based on a distributed X-ray source imaging system according to an exemplary embodiment of the present disclosure.

According to yet another aspect of the inventive concept of the present disclosure, a coordinate calibration method based on a distributed X-ray source imaging system is provided, which is applicable to the coordinate calibration device described in the above embodiments. As shown in FIG. 9, the coordinate calibration method includes step S01 to step S04.

According to some embodiments of the present disclosure, in step S01, a relative position of at least one of the detecting portion 1 and the X-ray source 2 with respect to the first marker portion 3 is determined.

According to some embodiments of the present disclosure, in step S02, the detecting portion 1 is fixed in a target region so that the detecting portion 1 and the X-ray source 2 are respectively located on opposite sides of the object to be detected. Optionally, the object to be detected is a tooth or a limb joint.

According to some embodiments of the present disclosure, in step S03, X-rays emitted from the X-ray source 2 are directed to the detecting portion 1.

According to some embodiments of the present disclosure, in step S04, a coordinate calibration is performed on image data acquired by the detecting portion 1 according to the relative position data of at least one of the detecting portion 1 and the X-ray source 2 with respect to the first marker points 4 on the first marker portion 3.

According to some embodiments of the present disclosure, the first marker points 4 have three-dimensional geometric shapes. By using the shapes of the first marker points 4 and the imaging coordinates of the first marker points 4, the spatial coordinates of the X-ray source 2 and the detecting portion 1 may be calculated to obtain the relative positional relationship between the X-ray source 2 and the detecting portion 1.

It should be noted that in this embodiment, the first marker portion 3 may be bound to the detecting portion 1, or the first marker portion 3 may be bound to the X-ray source 2.

According to some embodiments of the present disclosure, step S01 may further include fixing the detecting portion 1 in the target region, so that the detecting portion 1 and the first marker portion 3 are located on the same side of the object to be detected, and the object to be detected, the first marker portion 3, and the detecting portion 1 are sequentially arranged in close contact.

In an embodiment of the present disclosure, step S01 may further include fixing the detecting portion 1 in the target region, so that the first marker portion 3 and the detecting portion 1 are respectively located on opposite sides of the object to be detected. According to some embodiments of the present disclosure, the relative positional relationship between the X-ray source 2 and the detecting portion 1 is represented by a functional relationship between spatial coordinates of focal spots of the X-ray source 2 and spatial coordinates of pixels of the detecting portion 1.

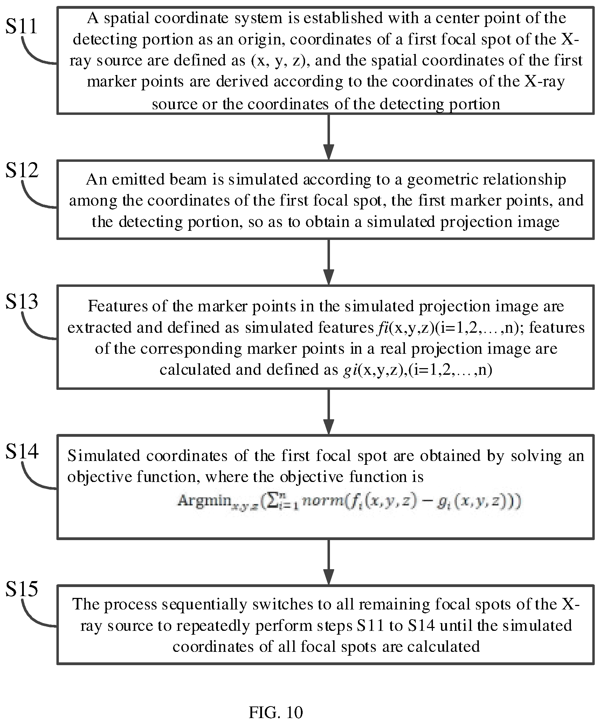

FIG. 10 shows a flowchart of steps for obtaining focal spots of the X-ray source in the coordinate calibration method based on a distributed X-ray source imaging system according to an exemplary embodiment of the present disclosure.

According to some embodiments of the present disclosure, the principle of the calibration method includes establishing a spatial coordinate system with a center point of the detecting portion 1 as an origin and solving the coordinates of all focal spots of the distributed X-ray source. Specifically, as shown in FIG. 10, the step of obtaining focal spots of the X-ray source 2 includes step S11 to step S15.

According to some embodiments of the present disclosure, in step S11, a spatial coordinate system is established with a center point of the detecting portion 1 as an origin, coordinates of a first focal spot of the X-ray source 2 are defined as (x, y, z), and the spatial coordinates of the first marker points 4 are derived according to the coordinates of the X-ray source 2 or the coordinates of the detecting portion 1.

According to some embodiments of the present disclosure, in step S12, an emitted beam is simulated according to a geometric relationship among the coordinates of the first focal spot, the first marker points 4, and the detecting portion 1, so as to obtain a simulated projection image.

According to some embodiments of the present disclosure, in step S13, features of the marker points in the simulated projection image are extracted and defined as simulated features *fᵢ*(*x, y,* z),(i=1,2,...,n); features of the corresponding marker points in a real projection image are calculated and defined as *gᵢ*(*x, y,* z),(i=1,2,...,n). Optionally, the features of the marker points include coordinates of the center points of the edges of the marker points, the centroid coordinates of the marker points, and the like.

According to some embodiments of the present disclosure, in step S14, simulated coordinates of the first focal spot are obtained by solving an objective function, where the objective function is as follows: ${Argmin}_{x , y , z} \left({\sum }_{i = 1}^{n} norm \left({f}_{i}\left(x, y, z\right)-{g}_{i}\left(x, y, z\right)\right)\right)$ where *norm*() represents a norm for calculating the difference between features, which may be selected from L2 norm, L1 norm, infinity norm, and the like; and Argmin represents minimizing the objective function.

According to some embodiments of the present disclosure, in step S15, the process sequentially switches to all remaining focal spots of the X-ray source 2 to repeatedly perform steps S11 to S14 until the simulated coordinates of all focal spots are calculated.

According to some embodiments of the present disclosure, in step S15, since the actual geometric relationships among the focal spots are known, taking a linear arrangement of focal-spot coordinates as an example, the simulated coordinates are fitted (by least squares method, etc.), and the coordinates of the point on the fitted line closest to the simulated coordinates of the focal spot are then taken as the focal-spot coordinates.

According to some embodiments of the present disclosure, prior to intraoral dental tomography or limb joint imaging, the detecting portion module is installed as required. The cubic first marker points 4 may be made of an X-ray transmissive material and placed in a region that does not affect the imaging quality of the target tooth/limb joint. The main function of the first marker portion 3 is to fix the positional relationship between the first marker points 4 and the detecting portion 1, and it is preferably arranged so that it does not affect the imaging quality of the target tooth/limb joint.

According to some embodiments of the present disclosure, when the first marker points 4 are installed between the tooth and the detecting portion 1, the first marker points 4 are embedded into the first marker portion 3, and the first marker portion 3 is tightly attached to the surface of the detecting portion 1. The first marker portion may be detachable or non-detachable. A non-detachable first marker portion 3 and the detecting portion 1 are bound and inseparable; a detachable first marker portion 3 may be freely removed from and installed onto the detecting portion 1, so that a new detecting portion module may be formed as required.

According to some embodiments of the present disclosure, when the first marker points 4 are installed between the tooth and the X-ray source 2, the first marker portion 3 and the detecting portion 1 are connected by a rigid support. In this case, the distributed point source and the detecting portion module may be mutually connected by the rigid support, or may be separated so that the point source is in a relatively free state.

According to some embodiments of the present disclosure, after the phantom is installed, the point sources emit X-rays in sequence, and the coordinates of the detecting portion 1 relative to the point sources are determined according to the features of the first marker points 4 on the projection image. Thereafter, three-dimensional tomographic imaging may be performed.

Thus, embodiments of the present disclosure have been described in detail with reference to the accompanying drawings. It should be noted that implementations not shown or described in the drawings or the specification are those well known to persons skilled in the art and are not described in detail herein. In addition, the definitions of the above-mentioned components are not limited to the specific structures, shapes, or forms mentioned in the embodiments. Those of ordinary skill in the art may make simple changes or substitutions thereto.

It should also be noted that in the specific embodiments of the present disclosure, unless otherwise expressly indicated to the contrary, the numerical parameters described in the specification and the appended claims are approximate values, which may vary depending on the desired characteristics obtained through the teachings of the present disclosure. Specifically, all numerical values used to describe dimensions, ranges, conditions, etc. in the specification and claims should be understood to be modified in all instances by the term "about". In general, this means that the values include variations of ±10% in some embodiments, ±5% in some embodiments, ±1% in some embodiments, and ±0.5% in some embodiments, from the specified quantity.

Those skilled in the art will appreciate that the features described in various embodiments and/or claims of the present disclosure may be combined and/or associated in multiple ways, even if such combinations or associations are not explicitly described herein. In particular, without departing from the spirit and teaching of the present disclosure, the features described in various embodiments and/or claims of the present disclosure may be combined and/or associated in multiple ways. All such combinations and/or associations fall within the scope of the present disclosure.

The foregoing specific embodiments further illustrate the objectives, technical solutions, and beneficial effects of the present disclosure. It should be understood that the above description is merely illustrative of specific embodiments of the present disclosure and is not intended to limit the present disclosure. Any modifications, equivalent substitutions, or improvements made within the spirit and principles of the present disclosure shall fall within the scope of protection of the present disclosure.

## Claims

1. A coordinate calibration device based on a distributed X-ray source imaging system, comprising:
an X-ray source configured to emit X-rays; and
a detector, comprising:
a detecting portion configured to be disposed on a side of an object to be detected opposite to the X-ray source, wherein the detecting portion is configured to receive the X-rays to image the object to be detected; and
a first marker portion disposed between the detecting portion and the X-ray source, wherein the first marker portion comprises a region of interest and a non-region of interest outside the region of interest, an image of the object to be detected after exposure is located in the region of interest, the first marker portion is provided with a plurality of first marker points having three-dimensional geometric shapes, and images of the plurality of first marker points after exposure are located in the first marker portion for a coordinate calibration of X-ray imaging;
wherein the coordinate calibration comprises calculating a relative positional relationship between the detecting portion and the X-ray source by using the shapes of the plurality of first marker points and imaging shapes of the plurality of first marker points, and the coordinate calibration is performed using the same dataset with X-ray scanning and three-dimensional imaging of the target object.

2. The coordinate calibration device of claim 1, wherein each of the first marker points comprises a marker symbol or a marker pattern, and different marker symbols or marker patterns have a predetermined relative positional relationship.

3. The coordinate calibration device of claim 1, wherein the image of at least one first marker point after exposure is located in the non-region of interest for the coordinate calibration of X-ray imaging; and/or
wherein the image of at least one first marker point after exposure is located in the region of interest for the coordinate calibration of X-ray imaging.

4. The coordinate calibration device of claim 1, wherein the first marker points are dot-shaped, filament-shaped, strip-shaped, L-shaped columns, or S-shaped columns.

5. The coordinate calibration device of claim 1, wherein the number of the first marker points is less than or equal to four.

6. The coordinate calibration device of claim 1, wherein the first marker points are embedded into a top portion of the first marker portion on a side away from the detecting portion.

7. The coordinate calibration device of claim 6, wherein the detecting portion and the first marker portion are located on the same side of the object to be detected, and the object to be detected, the first marker portion, and the detecting portion are sequentially disposed in close contact.

8. The coordinate calibration device of claim 7, further comprising:
a first support having one end connected to the detecting portion and the other end connected to the X-ray source, wherein the first support is a rigid structure configured to maintain the relative positional relationship between the detecting portion and the X-ray source unchanged.

9. The coordinate calibration device of claim 1, further comprising:
a second support having one end connected to the detecting portion and the other end connected to the first marker portion, wherein the detecting portion and the first marker portion are respectively disposed on opposite sides of the object to be detected, and the second support is a rigid structure configured to maintain a relative positional relationship between the detecting portion and the first marker portion unchanged.

10. The coordinate calibration device of claim 9, wherein the other end of the second support extends in a direction away from the detecting portion and is connected to the X-ray source, and the second support is configured to maintain a relative positional relationship among the X-ray source, the detecting portion, and the first marker portion unchanged.

11. The coordinate calibration device of claim 1, wherein the first marker portion and the detecting portion are respectively located on opposite sides of the object to be detected, and the coordinate calibration device further comprises:
a third support having one end connected to the first marker portion and the other end connected to the X-ray source, wherein the third support is a rigid structure configured to maintain a relative positional relationship between the first marker portion and the X-ray source unchanged.

12. The coordinate calibration device of claim 11, further comprising:
a second marker portion disposed between the detecting portion and the object to be detected, wherein opposite sides of the second marker portion are respectively in close contact with the object to be detected and the detecting portion, and a second marker point is embedded into a top portion of the second marker portion on a side away from the detecting portion.

13. The coordinate calibration device of claim 11, further comprising:
a second marker portion disposed on a side of the object to be detected opposite to the detecting portion, wherein a second marker point is embedded into a top portion of the second marker portion on a side away from the detecting portion; and
a fourth support having one end connected to the detecting portion and the other end connected to the second marker portion, wherein the fourth support is a rigid structure configured to maintain a relative positional relationship between the detecting portion and the second marker portion unchanged.

14. The coordinate calibration device of claim 1, wherein a thickness of the first marker portion and a thickness of the second marker portion range from 1 mm to 3 mm or from 2 mm to 15 mm.

15. The coordinate calibration device of claim 1, wherein the X-ray source is a distributed multi-point source array.

16. The coordinate calibration device of claim 1, wherein:
both ends of the first support are respectively connected to the detecting portion and the X-ray source by magnetic attraction; and/or
both ends of the second support are respectively connected to the detecting portion and the first marker portion by magnetic attraction; and/or
both ends of the third support are respectively connected to the first marker portion and the X-ray source by magnetic attraction; and/or
both ends of the fourth support are respectively connected to the detecting portion and the second marker portion by magnetic attraction.

17. The coordinate calibration device of claim 1, wherein the relative positional relationship between the detecting portion and the X-ray source comprises a tilt angle and an offset angle of the detecting portion relative to the X-ray source as well as a distance between the detecting portion and the X-ray source.

18. The coordinate calibration device of claim 1, wherein the object to be detected is a tooth, a torso, or a whole human body.

19. An imaging system, comprising:
the coordinate calibration device of any one of claims 1 to 18;
a data conversion device configured to convert a detection signal generated by a detecting portion of the coordinate calibration device into digital data; and
an image processing device configured to perform imaging according to the digital data.

20. A coordinate calibration method based on a distributed X-ray source imaging system, applicable to the coordinate calibration device of any one of claims 1 to 18, the coordinate calibration method comprising:
step S01 of determining a relative position of at least one of a detecting portion and an X-ray source with respect to a first marker portion;
step S02 of fixing the detecting portion in a target region so that the detecting portion and the X-ray source are located on opposite sides of an object to be detected;
step S03 of directing X-rays emitted from the X-ray source to the detecting portion; and
step S04 of performing a coordinate calibration on image data acquired by the detecting portion according to relative positional data of at least one of the detecting portion and the X-ray source with respect to the first marker points on the first marker portion;
wherein the first marker points have three-dimensional geometric shapes, and spatial coordinates of the X-ray source and spatial coordinates of the detecting portion are calculated using the shapes of the first marker points and imaging coordinates of the first marker points, to obtain a relative positional relationship between the X-ray source and the detecting portion.

21. The coordinate calibration method of claim 20, wherein step S01 comprises:
fixing the detecting portion in the target region, so that the detecting portion and the first marker portion are located on the same side of the object to be detected, and the object to be detected, the first marker portion, and the detecting portion are sequentially disposed in close contact; or
fixing the detecting portion in the target region, so that the first marker portion and the detecting portion are respectively located on opposite sides of the object to be detected.

22. The coordinate calibration method of claim 20, wherein the relative positional relationship between the X-ray source and the detecting portion is represented by a functional relationship between spatial coordinates of focal spots of the X-ray source and spatial coordinates of pixels of the detecting portion.

23. The coordinate calibration method of claim 22, wherein the focal spots of the X-ray source are obtained by:
step S11 of establishing a spatial coordinate system with a center point of the detecting portion as an origin, defining coordinates of a first focal spot of the X-ray source as (x, y, z), and deriving spatial coordinates of the first marker point according to coordinates of the X-ray source or coordinates of the detecting portion;
step S12 of simulating an emitted beam according to a geometric relationship among the coordinates of the first focal spot, the first marker points, and the detecting portion, to generate a simulated projection image;
step S13 of extracting features of the first marker points in the simulated projection image, defined as simulated features *fᵢ*(*x,y,z*) ,(i=1,2,...,n); and calculating features of the corresponding first marker points in a real projection image, defined as *gᵢ*(*x, y, z*)*,*(i=1,2,*...,*n);
step S14 of obtaining simulated coordinates of the first focal spot by solving an objective function, wherein the objective function is: ${Argmin}_{x , y , z} \left({\sum }_{i = 1}^{n} norm \left({f}_{i}\left(x, y, z\right)-{g}_{i}\left(x, y, z\right)\right)\right) ; and$and
step S15 of sequentially switching to all remaining focal spots of the X-ray source, and repeatedly performing steps S11 to S14 until the simulated coordinates of all focal spots are calculated.
